Europäisches Patentamt

European Patent Office

Office européen des brevets

(19)

(11) Publication number: **0 017 441**
**B2**

(12)

# NEW EUROPEAN PATENT SPECIFICATION

(45) Date of publication of the new patent specification:
28.10.87

(21) Application number: 80300978.6

(22) Date of filing: 28.03.80

(51) Int. Cl.⁴: **C 07 C 51/14**, C 07 C 53/122,
C 07 C 53/128, C 07 C 51/377,
C 07 C 57/04, C 07 C 67/38

(54) Preparation of esters.

(30) Priority: 09.04.79 US 28459
09.04.79 US 28460

(43) Date of publication of application:
15.10.80 Bulletin 80/21

(45) Publication of the grant of the patent:
03.08.83 Bulletin 83/31

(45) Mention of the opposition decision:
28.10.87 Bulletin 87/44

(84) Designated Contracting States:
AT BE CH DE FR GB IT LU NL SE

(56) References cited:
DE - A - 1 064 941
DE - A - 1 085 143
DE - A - 1 202 265
DE - A - 2 630 235
FR - A - 1 377 834
GB - A - 1 250 479
US - A - 2 135 459

CHEMICAL ABSTRACTS, vol. 84, no. 3, January 19, 1976,
page 409, column 1, abstract no. 16702a COLUMBUS,
Ohio (US) P.P. GUSHCHIN et al.: "Synthesis of
branched carboxylic acids from technical fractions of
olefins, carbon monoxide, and water in the presence of
complex boron fluoride compounds"
K.E. Möller, Brennstoff-Chemie,Vol.47, p. 10 -15 (1966)

(73) Proprietor: CHEM SYSTEMS, Inc., 747 Third Avenue,
New York New York 10017 (US)

(72) Inventor: Jung, John A., 2 Coolidge Avenue, East
Hanover Morris New Jersey 07936 (US)
Inventor: Peress, Jimmy, 15 Alexander Drive, West
Haven New Haven Connecticut 06516 (US)
Inventor: Gelbein, Abraham P., 925 Woodland Avenue,
Plainfield Union, New Jersey 07060 (US)

(74) Representative: Harrison, Michael Robert et al,
URQUHART-DYKES & LORD 5th Floor Tower House
Merrion Way, Leeds LS2 8PA West Yorkshire (GB)

EP 0 017 441 B2

ACTORUM AG

## Description

This invention relates to a method for preparing carboxylic esters from olefins.

US-A 2 135 459 discloses a process for the carbonylation of olefins utilising a catalyst comprising one mol of water per mol of boron fluoride at temperatures in excess of 160°C and at high pressure.

DE-A 1 202 265 discloses the carbonylation of olefins using a catalyst comprising boron fluoride and water in which the molar ratio of boron fluoride to water is between about 1 and 2.5 with a ratio of 2 being preferred. Although this process appears to give good results for olefins having from 8 to 10 carbon atoms, much less good results are obtained with ethylene and propylene.

According to the present invention there is provided a process for the carbonylation of an olefin by reaction of the olefin with carbon monoxide in the presence of a catalyst characterised in that the olefin is ethylene or propylene and the catalyst contains equimolar amounts of $BF_3$ and an alcohol and the reaction being conducted at a temperature of from 0°C to 100°C, whereby the product obtained is a carboxylate ester wherein the carboxylate group has one more carbon atom than the olefin employed.

Accordingly it has been surprisingly discovered that the use of the specified catalyst system in which equimolar amounts of boron fluoride and the alcohol are used, gives high olefin conversion rates at low reaction temperatures.

The catalyst is in the form of a complex of $BF_3$ and an alcohol. It is a stable complex having specific physical properties. It exists as a liquid at room temperature and thereafter can be conveniently used as a reaction solvent.

The active catalyst complex is formed by the addition of one mole of $BF_3$ to one mole of the alcohol. The reaction takes place in the liquid catalyst solution through which gaseous carbon monoxide and the olefin are passed. The reaction conditions are moderate and a high yield of the ester is obtained. The carboxylic acid portion of the ester product has one more carbon atom than the initial olefin. Substantially no polymerization of the olefin occurs. The saturated carboxylic acid ester product may be readily dehydrogenated to form an unsaturated carboxylic acid ester, e.g. methyl isobutyrate obtained from the carbonylation of propylene may be dehydrogenated to form methyl methacrylate.

Alternatively, the propylene may be carbonylated to form isobutyric acid which may thereafter be dehydrogenated to methacrylic acid.

In a preferred embodiment in accordance with the present invention, the reaction is carried out until about 50% of the alcohol in said catalyst is consumed to form a carboxylic acid ester and liberate free $BF_3$; sufficient $BF_3$ is separated from the reaction mass to leave a first residue containing equimolar amounts of the $BF_3$, the alcohol and the carboxylic acid ester; the first residue is admixed with additional alcohol; thereafter said admixture is distilled to separate overhead an azeotrope of said alcohol and said carboxylic acid ester until a second residue containing $BF_3$ to alcohol in a molar ratio of 1:2 remains; thereafter said second residue is combined with additional $BF_3$ to form the catalyst complex. In this way the $BF_3$ alcohol catalyst is recovered and recycled in a convenient and economical manner. In this embodiment, after the carbonylation the reaction mass consisting of the carboxylic acid ester, the alcohol, and the $BF_3$ in a molar ratio of 1:1:2 is subjected to a distillation step wherein one-half of the $BF_3$ is removed as a distillate fraction. The bottoms product from this step consists of the same components in a molar ratio 1:1:1. This mixture is then further distilled after sufficient additional alcohol is added to form a low boiling azeotrope with all of the carboxylic acid ester. This azeotrope is recovered as an overhead product. The residue material remaining is a $BF_3$/alcohol 1:2 complex. This material may be easily reconstituted to form the 1:1 complex catalyst by the addition of free $BF_3$, the latter of which may be that initially separated from the reaction mass.

In the present invention, gaseous feedstocks consisting of either propylene or ethylene and carbon monoxide are used. The olefins may be obtained from any source, most generally from steam cracking of hydrocarbons.

The carbon monoxide employed should have a purity of at least 99%, though mixtures of carbon monoxide and other inert gases, such as carbon dioxide, may be used.

It is preferred that the olefin and carbon monoxide be of high purity since this will simplify product recovery and minimize losses in purge streams required to remove inerts from the reaction system.

The reaction may be carried out at temperatures of from 0°C to 100°C, preferably from 20°C to 60°C. The temperatures should be kept at moderate levels throughout the process because high temperatures could result in the formation of byproducts, especially heavier esters.

In the carbonylation reaction of the invention, one mole of carbon monoxide reacts with each mole of olefin. This is known as the external reactant ratio. On the other hand, it is desirable to maintain a large molar excess of carbon monoxide in the vapor phase in order to suppress undesirable side reactions. This ratio (known as the internal carbon monoxide to olefin molar ratio) is controlled by the system pressure, degree of agitation, and purge rate and is broadly at least 5:1, preferably at least 8:1. As a practical matter, ratios of not more than 1000:1, preferably not more than 100:1, are used.

In view of the foregoing, it will be understood that in a batchwise process and during the start-up of a continuous process a large molar excess of the carbon monoxide is fed. However, in the continuous process, once steady state conditions are achieved, only about one mole of carbon monoxide is fed to the reactor for each mole of olefin.

The reaction pressure, while not critical, is generally of from $1 \times 10^6$ to $3 \times 10^7$ Pa (10 to 30 atmospheres), most preferably from $3 \times 10^6$ to $1 \times 10^7$ Pa (30 to 100). While higher pressures are not detrimental and in some instances actually favor selectivity to the desired products, again practical considerations such as equipment design and safety factors favor the use of the pressure ranges set forth above.

The selection of the appropriate catalyst complex is an essential feature of the invention. As pointed our previously, in the prior art the most commonly used catalyst complex contains one mole of $BF_3$ for each two moles of water or alcohol. In contrast, the catalyst complex used in the instant invention contains equal molar amounts of $BF_3$ and alcohol (hereinafter the "second component"). These catalysts are stable complexes having specific physical properties. They exist as liquids at room temperature and therefore can be conveniently used as the reaction solvent.

While it is understood that the 1:1 molar ratio catalyst is the active constituent in the instant invention, the catalyst may be prepared using ratios of from about 0.75 to 10 moles of $BF_3$ for each mole of the second component, preferably from .75 to 2 moles per mole. It will be understood that, when less than one mole of the $BF_3$ is utilized with, say, methanol, the catalyst is a mixture of $BF_3 \cdot CH_3OH$ and $BF_3 \cdot 2CH_3OH$. This latter compound is also a stable complex; however, in contrast to the 1:1 molar ratio catalyst, it is non-selective to the desired product and of relatively low activity. Accordingly, a substantial amount of such complex is undesirable.

On the other hand, where the molar ratio is in excess of 1:1, the 1:1 catalyst complex (e.g., $BF_3 \cdot CH_3OH$) is in admixture with uncomplexed $BF_3$. Since excess $BF_3$ is not catalytically active for the desired acid or ester, sizeable excesses are of little advantage.

As noted above, in performing the process of the invention it is advantageous to use the catalyst as the reaction medium. Other organic constituents may be present, so long as they do not interfere with the carbonylation. The reaction period is not critical, but should be selected so as to achieve acceptable conversions without unduly lengthening the process cycle. As a practical matter, the reaction period ranges from about 10 minutes to 3 hours.

Generally, the lower alkyl alcohols having from 1 to 4 carbon atoms are preferred as the second component. These include methanol, ethanol, propanol, isopropanol and n-butanol and its isomers. Additionally, other alcohols can be used. These include alkyl alcohols having from 5 to 12 carbon atoms and aralkyl alcohols such as benzyl alcohol, alpha-phenethyl alcohol and betaphenethyl alcohol. Methanol is the most useful.

In addition, it has been found that other catalyst additives such as hydrogen fluoride, sulphuric acid, and phosphoric acid, are not necessary for the synthesis of the dessired products.

The catalyst recovery embodiment of the invention may be described using the preparation of methyl isobutyrate as an example. Initially, the carbonylation is conducted until one-half of the methanol is consumed in the formation of the methyl isobutyrate products. The resulting product mixture has a composition consisting of methyl isobutyrate, methanol and $BF_3$ in a molar ratio of 1:1:2.

This reaction mass is stripped in a countercurrent contacting tower to separate half the $BF_3$ contained therein as a vapor over-head product and to produce a residue product containing equimolar amounts of methyl isobutyrate methanol and $BF_3$ (hereinafter the "1:1:1 complex"). Operating conditions for this step depend on the vapor-liquid equilibria for the system and can be varied over a wide range of temperatures and pressures. The separation is conveniently done at ambient pressure and at approximately 85°C, the boiling point of the 1:1:1 complex. Under these conditions, the stripping action is provided by the boiling 1:1:1 complex vapors which are generated in the bottom of the stripping column. These vapors flow countercurrently to the reaction mixture feed which is fed at the top of the column. Alternatively, the column can be operated at a temperature and pressure wherein the 1:1:1 complex does not boil by using an inert stripping gas, e.g., nitrogen introduced at the bottom of the column. Generally, it is preferred to maintain the column temperature below 100°C to minimize byproduct formation.

After the separation of the $BF_3$, the residue contained equimolar quantities of $BF_3$, methanol and the methyl isobutyrate. The methyl isobutyrate and the $BF_3$ contained in this residue from the stripping step cannot readily be recovered by means know to the art without destroying the catalyst complex. For example, $BF_3$ or the organic components cannot be preferentially stripped from this complex, nor are there any known extraction techniques which will preferentially extract the $BF_3$ or the organic components. Methods known to the art for making the separation involve reaction of the $BF_3$ with another component, e.g., water or sodium chloride. In the first case hydrolysis of the $BF_3$ occurs with release of the organic components. In the second case a $BF_3$/sodium chloride compound is formed with release of the organic components. However, there is no known practical method for recovering the $BF_3$ from the $BF_3$ hydrolysis compounds or the $BF_3$/sodium chloride compound.

In accordance with the invention, to separate the desired components, methanol is added to the 1:1:1 complex, preferably in a distillation zone. The amount of methanol added corresponds to at least that required to produce a methyl isobutyrate/methanol azeotrope as an overhead product and a bottoms product consisting of a complex of $BF_3/CH_3OH$ in 1:2 molar ratio. The composition of this azeotrope, at an atmospheric pressure, is 75 weiht percent methanol, 25 weight percent methyl isobutyrate. This is equivalent to a methanol/methyl isobutyrate molar ratio of 9.56. Thus, if the distillation is conducted at ambient pressure at least 10.56 additional moles of methanol must be added to the distillation zone. Technically there is no upper limit to the amount of methanol that can be added. The amount that is added is dictated by the design of the distillation equipment wherein energy requirements are balanced against equipment costs.

The separation is conveniently carried out in a conventional continuous distillation column containing trays or packing. The 1:1:1 complex is generally fed near the middle of the column with the methanol added above this feed point. The distillate fraction from this column is a low boiling methanol/methyl isobutyrate azeotrope and any additional uncomplexed methanol. The bottoms product is a $BF_3$/methanol complex in 1:2 molar ratio. Operating temperatures and pressures for the column are a function of the vapor pressure-temperature curve for the complex since this is the material boiling in the reboiler. Typical pressure-temperature data is given below:

| Temperature, °C | Vapor pressure, Pa (mm Hg) |
| --- | --- |
| 57 | 533 (4) |
| 130 | 13332 (100) |
| 200 | 101325 (760) |

Preferred operating temperatures are from 50 to 200°C. These temperatures minimize side reactions which can lead to yield losses and complicate subsequent purification steps.

The $BF_3$/methanol 1:2 bottoms product from the column is combined preferably with the $BF_3$ originally separated from the reaction mass and reused in the carbonylation. If necessary, of course, makeup material may be added to this stream.

The methyl isobutyrate and methanol may be separated by any conventional means. In one particular technique, azeotropic distillation is used wherein a liquid paraffin, preferably having a boiling point of 30°C to 150°C, is added to form a low boiling azeotrope with the methanol. The ambient pressure boiling point of the octane/methanol azeotrope is approximately 65°C, while the methyl isobutyrate has an ambient pressure boiling point of approximately 90°C. The azeotrope is removed overhead and condensed. This condensate is then mixed with water in a separate vessel wherein an aqueous methanol phase and a paraffin phase, essentially free of methanol, are formed. The organic phase is recycled to the azeotropic distillation column. The aqueous methanol phase is then further distilled to recover methanol as an overhead product for recycle to the aforementioned $CH_3OH$/methyl isobutyrate column.

The products of the carbonylation may be dehydrogenated by several known procedures such as described in Japan Kokai 78 82,720 or Japan 73 19,614 where these carbonylation products (methyl isobutyrate or isobutyric acid) are oxidatively dehydrogenated at 300°C and $10^5$ Pa (1 atm.) pressure with oxygen-cotaining gases over catalysts composed of mixed metal oxides, the major component being molybenum oxide. In U.S. Patent 3 721 705, isobutyric acid or methyl isobutyrate is oxidatively dehydrogenated at 500°C in the presence of sulfur. In British Patent 1 141 625, a dehydrogenation is carried out without added oxidizing agents over alumina catalysts at 600°C and reduced pressure.

To illustrate more fully the instant invention, attention is directed towards the following examples:

Example 1

To a 600 ml stirred autoclave at 20°C is added 100 g of a $BF_3 \cdot CH_3OH$ catalyst. A 9:1 carbon monoxide/propylene mixture is added to the autoclave at $6 \times 10^6$ Pa (60 atm). The mixture is heated to 50°C and held at this temperature for one hour. A sample taken from the autoclave is analyzed by gas-liquid chromatography. All of the propylene is converted and the selectivity to methyl isobutyrate is 94%. The autoclave is cooled, depressurized, and then repressurized again to $6 \times 10^6$ Pa (60 atm) with the 9:1 gas mixture. This procedure is repeated several times until about 50% of the methanol in the catalyst reacts. The mixture remaining in the autoclave is again analyzed and found to be approximately a 2:1:1 mixture of boron trifluoride/methanol/methyl isobutyrate (about 38 wt. % isobutyrate). The selectivity to the methyl isobutyrate is 88%.

The catalyst recovery embodiment of the subject invention is illustrated by treating the reaction mass in accordance with the following sequence: The reaction mixture is introduced into a distillation flask wherein the liberated $BF_3$ is removed overhead at a temperature of 60°C and a pressure of $1.33 \times 10^4$ Pa (100 mm Hg). This $BF_3$ is recycled to the autoclave for reuse as hereinafter described.

This leaves in the distillation flask a solution containing a 1:1:1 ratio $BF_3$/methanol/methyl isobutyrate complex. To this, sufficient methanol is added to convert the solution to 1:10:1 molar ratio. The resulting solution is distilled at a pressure of $1.33 \times 10^3$ Pa (10 mm Hg) until the overhead temperature reaches 80°C to separate a methyl isobutyrate/methanol azeotrope. Continued distillation at 80°C removes additional methanol. Analysis of the materials remaining in the distillation flask shows the presence of a 1:2 $BF_3$/methanol complex. This latter complex is recycled to the carbonylation reactor with the $BF_3$ initially stripped from the distillation flask.

Octane is added to the methyl isobutyrate/methanol solution and a low boiling azeotrope of methanol and octane is distilled overhead. The residue is methyl isobutyrate. It may be dehydrogenated to form methyl methacrylate.

The methyl isobutyrate thus obtained is dehydrogenated to form methyl methacrylate in accordance with the following procedure:

Methyl isobutyrate, oxygen, steam and nitrogen in a molar ratio of 3:4:4:89 are fed into a reactor containing a catalyst consisting of molybdenum, vanadium and phosphorous oxides in an atomic ratio of Mo:V:P of 12:2:1. The temperature is maintained at 300°C with a residence time of 1 second. Under these conditions 90% of the feed methyl isobutyrates is converted to methyl methacrylate and methacrylic acid with a combined selectivity of 75%. The methacrylic acid is then esterified to methyl methacrylate.

A direct method of recovering the catalyst components has been attempted but not found successful. For example, a molar excess of sodium chloride was added to the admixture of $BF_3$/methanol/methyl isobutyrate 1:1:1 complex in an attempt to form a complex of the $BF_3$ with the sodium chloride and separate the complex. In this procedure, from the mixture thus formed the methyl isobutyrate and methanol were easily separated by distillation. However, the $BF_3$/sodium chloride complex salt, even after heating to 400°C, did not completely break down into its component salts. Analysis of the $BF_3$ thus recovered indicates that only 50% of the initial $BF_3$ present was recovered.

Still another approach to separating the 1:1:1 complex was to use n-heptane to selectively extract the methyl isobutyrate. Only about 10% of the methyl isobutyrate was extracted into the heptane after extraction with one volume of the heptane for two volumes of the complex.

In still another approach, an admixture of $BF_3$/methanol/methyl isobutyrate in a molar ratio of 1:1:0.5 was introduced to the top of a countercurrent stripping column while nitrogen was passed through the bottom of the column under atmospheric pressure at a temperature of 80°C. The effort to preferentially strip the $BF_3$ was unsuccessful. Analysis showed that no $BF_3$ was removed by this technique.

Example 2

In this example, 90 g of a $BF_3$ $CH_3OH$ complex is added to a 600 ml autoclave and maintained at 20°C. A 9:1 carbon monoxide/ ethylene gas mixture is added 6.8 × 10⁶ Pa (68 atms). After addition of this mixture, the autoclave is heated to 50°C and maintained at this temperature for three hours. The autoclave is cooled and depressurized. Analysis by gas-liquid chromatography indicates that the autoclave contents consist of 1 to 2% by weight methyl propionate plus 2 to 3% by weight of heavier materials. The selectivity to methyl propionate is about 25%. Under the conditions of the oxidative dehydrogenation described in Example 1, methyl propionate gives a combined selectivity of 75% to methyl acrylate and acrylic acid.

Example 3

Under conditions similar to Example 2, except that $2BF_3 \cdot HC_3OH.H_2O$ is the catalyst, ethylene is carbonylated to produce propionic acid in 25% selectivity. Propionic acid is dehydrogenated in accordance with dehydrogenation conditions of Example 1 and gives acrylic acid in 80% selectivity.

Example 4

Under the conditions of Example 1, except that $BF_3 \cdot i\text{-}C_3H_7OH$ is the catalyst, propylene is carbonylated to give isopropyl isobutyrate in 90% selectivity.

Comparative Example I

The procedure of Example 1 is repeated, except that 100 g of $BF_3.2CH_3OH$ is employed. Only 10 to 20% of the propylene is converted in one hour. The only product produced is isopropyl methyl ether. This clearly shows the criticality of the ratio of the $BF_3$ to the second component of the catalyst.

**Claims**

1. A process for the carbonylation of an olefin by reaction of the olefin with carbon monoxide in the presence of a catalyst characterised in that the olefin is ethylene or propylene and the catalyst contains equimolar amounts of $BF_3$ and an alcohol and the reaction being conducted at a temperature of from 0°C to 110°C, whereby the product obtained is a carboxylate ester wherein the carboxylate group has one more carbon atom than the olefin employed.

2. A process according to claim 1 characterised in that the reaction is carried out until about 50% of the alcohol in said catalyst is consumed to form a carboxylic acid ester and liberate free $BF_3$; sufficient $BF_3$ is separated from the reaction mass to leave a first residue containing equimolar amounts of the $BF_3$, the alcohol and the carboxylic acid ester; the first residue is admixed with additional alcohol; thereafter said admixture is distilled to separate overhead an azeotrope of said alcohol and said carboxylic acid ester until a second residue containing $BF_3$ to alcohol in a molar ratio of 1:2 remains; and thereafter said second residue is combined with additional $BF_3$ to form the catalyst complex.

3. A process according to claim 2 characterised in that the additional $BF_3$ added to the second residue is that $BF_3$ initially removed from the reaction mass.

4. A process according to claim 2 or claim 3 characterised in that the carboxylic acid ester and alcohol stream is combined with an azeotroping solvent and said combination is subjected to further distillation wherein a low boiling azeotrope comprised of solvent and alcohol is removed from the carboxylic acid ester.

5. A process according to any one of claims 2 to 4 characterised in that the azeotroping solvent is a paraffin having a boiling point of from 30°C to 150°C.

6. A process according to claim 5 characterised in that the paraffin is octane.

7. A process according to any one of claims 2 to 6 characterised in that the catalyst is $BF_3 \cdot CH_3OH$, and the product obtained is methyl isobutyrate, where the olefin is propylene, or methyl propionate, where the olefin is ethylene.

8. A process according to any of the preceding claims characterised in that the catalyst is $BF_3 \cdot CH_3OH$ and is formed by reaction of from .75 to 2 moles of $BF_3$ with one mole of $CH_3OH$.

9. A process according to any of the preceding claims characterised in that the carbonylation reaction product is dehydrogenated to a corresponding unsaturated carboxylic acid ester.

10. A process for the carbonylation of an olefin by reaction of the olefin with carbon monoxide in the presence of a catalyst characterised in that the olefin is ethylene or propylene and the catalyst contains equimolar amounts of $BF_3$ and an alcohol and the reaction bein conducted at a temperature of from 0°C to 100°C, whereby the product obtained is a carboxylate ester wherein the carboxylate group has one more carbon atom than the olefin employed, the reaction being carried out until about 50% of the alcohol in said catalyst is consumed to form a carboxylic acid ester and liberate free $BF_3$; sufficient $BF_3$ is separated from the reaction mass to leave a first residue containing equimolar amounts of the $BF_3$, the alcohol and the carboxylic acid ester; the first residue is admixed with additional alcohol; thereafter said admixture is distilled to separate overhead an azeotrope of said alcohol and said carboxylic acid ester until a second residue containing $BF_3$ to alcohol in a molar ratio of 1:2 remains; and thereafter said second residue is combined with additional $BF_3$ to form the catalyst complex.

**Patentansprüche**

1. Verfahren zur Carbonylierung eines Olefins durch Umsetzung des Olefins mit Kohlenmonoxid in Gegenwart eines Katalysators, dadurch gekennzeichnet, dass das Olefin Ethylen oder Propylen ist und der Katalysator äquimolare Mengen von $BF_3$ und einem Alkohol enthält und dass die Umsetzung bei einer Temperatur von 0°C bis 100°C durchgeführt wird, wobei das erhaltene Produkt ein Carbonsäureester ist, in dem die Carboxylatgruppe ein Kohlenstoffatom mehr aufweist als das eingesetzte Olefin.

2. Verfahren nach Anspruch 1, dadurch gekennzeichnet, dass die Umsetzung durchgeführt wird, bis etwa 50% des Alkohols in dem Katalysator unter Bildung eines Carbonsäureesters und Entwicklung von freiem $BF_3$ verbraucht sind, dass von der Reaktionsmasse $BF_3$ in einer Menge abgetrennt wird, die ausreicht, um einen ersten Rückstand, der äquimolare Mengen des $BF_3$, des Alkohols und des Carbonsäureesters enthält, zurückzulassen, dass der erste Rückstand mit zusätzlichem Alkohol vermischt wird, dass diese Mischung danach destilliert wird, wobei über Kopf ein Azeotrop aus dem Alkohol und dem Carbonsäureester abgetrennt wird, bis ein zweiter Rückstand, der $BF_3$ und Alkohol in einem Molverhältnis von 1:2 enthält, verbleibt, und dass der zweite Rückstand danach zur Bildung des Katalysatorkomplexes mit zusätzlichem $BF_3$ vereinigt wird.

3. Verfahren nach Anspruch 2, dadurch gekennzeichnet, dass es sich bei dem zu dem zweiten Rückstand zugegebenen, zusätzlichen $BF_3$ um das anfänglich von der Reaktionsmasse abgetrennte $BF_3$ handelt.

4. Verfahren nach Anspruch 2 oder 3, dadurch gekennzeichnet, dass der Carbonsäureester und Alkohol enthaltende Strom mit einem azeotropbildenden Lösungsmittel vereinigt wird und dass der mit dem azeotropbildenden Lösungsmittel vereinigte Strom einer weiteren Destillation unterzogen wird, wobei von dem Carbonsäureester ein aus Lösungsmittel und Alkohol bestehendes, niedrigsiedendes Azeotrop abgetrennt wird.

5. Verfahren nach einem der Ansprüche 2 bis 4, dadurch gekennzeichnet, dass das azeotropbildende Lösungsmittel ein Paraffin mit einem Siedepunkt von 30°C bis 150°C ist.

6. Verfahren nach Anspruch 5, dadurch gekennzeichnet, dass das Paraffin Octan ist.

7. Verfahren nach einem der Ansprüche 2 bis 6, dadurch gekennzeichnet, dass der Katalysator $BF_3 \cdot CH_3OH$ ist und dass das erhaltene Produkt Methylisobutyrat ist, wenn das Olefin Propylen ist, oder Methylpropionat ist, wenn das Olefin Ethylen ist.

8. Verfahren nach einem der vorhergehenden Ansprüche, dadurch gekennzeichnet, dass der Katalysator $BF_3 \cdot CH_3OH$ ist und durch Umsetzung von 0,75 bis 2 mol $BF_3$ mit einem Mol $CH_3OH$ gebildet wird.

9. Verfahren nach einem der vorhergehenden Ansprüche, dadurch gekennzeichnet, dass das Produkt der Carbonylierungsreaktion unter Bildung eines entsprechenden ungesättigten Carbonsäureesters dehydriert wird.

10. Verfahren zur Carbonylierung eines Olefins durch Umsetzung des Olefins mit Kohlenmonoxid in Gegenwart eines Katalysators, dadurch gekennzeichnet, dass das Olefin Ethylen oder Propylen ist und der Katalysator äquimolare Mengen von $BF_3$ und einem Alkohol enthält, dass die Umsetzung bei einer Temperatur von 0°C bis 100°C durchgeführt wird, wobei das erhaltene Produkt ein Carbonsäureester ist, in dem die Carboxylatgruppe ein Kohlenstoffatom mehr aufweist als das eingesetzte Olefin, dass die Umsetzung durchgeführt wird, bis etwa 50% des Alkohols in dem Katalysator unter Bildung eines Carbonsäureesters und Entwicklung von freiem $BF_3$ verbraucht sind, dass von der Reaktionsmasse $BF_3$ in einer Menge abgetrennt wird, die ausreicht, um einen ersten Rückstand, der äquimolare Mengen des $BF_3$, des Alkohols und des Carbonsäureesters enthält, zurückzulassen, dass der erste Rückstand mit zusätzlichem Alkohol vermischt wird, dass diese Mischung danach destilliert wird, wobei über Kopf ein Azeotrop aus dem Alkohol und dem Carbonsäureester abgetrennt wird, bis ein zweiter Rückstand, der $BF_3$ und Alkohol in einem Molverhältnis von 1:2 enthält, verbleibt, und dass der zweite Rückstand danach zur Bildung des Katalysatorkomplexes mit zusätzlichem $BF_3$ vereinigt wird.

## Revendications

1. Procédé pour la carbonylation d'une oléfine par réaction de l'oléfine avec de l'oxyde de carbone en présence d'un catalysateur caractérisé en ce que l'oléfine est de l'éthylène ou du propylène et le catalysateur contient des quantités équimolaires de $BF_3$ et d'un alcool, et la réaction étant entreprise à une température de 0 à 100°C, ainsi le produit obtenue est un ester de carboxylate où le groupe carboxylate a un atome de carbone de plus que l'oléfine employée.

2. Procédé selon la revendication 1, caractérisé en ce que la réaction est effectuée jusqu'à ce qu'environ 50% de l'alcool dans ledit catalyseur soient consommés pour former un ester d'acide carboxylique et libérer $BF_3$ libre; suffisamment de $BF_3$ se sépare de la masse réactionnelle pour laisser un premier résidu contenant des quantités équimolaires de $BF_3$, de l'alcool et de l'ester d'acide carboxylique; le premier résidu est mélangé à de l'alcool supplémentaire; ensuite ledit mélange est distillé pour séparer en tête un azéotrope dudit alcool et dudit ester d'acide carboxylique jusqu'à ce qu'il reste un second résidu contenant $BF_3$ à l'alcool à un rapport molaire de 1:2; et ensuite ledit second résidu est combiné à du $BF_3$ supplémentaire pour former le complexe catalytique.

3. Procédé selon la revendication 2, caractérisé en ce que le $BF_3$ supplémentaire ajouté au second résidu est le $BF_3$ initialement retiré de la masse réactionnelle.

4. Procédé selon la revendication 2 ou la revendication 3, caractérisé en ce que le courant de l'ester de l'acide carboxylique et de l'alcool est combiné à un solvant rendant azéotrope et ladite combinaison est soumise à une autre distillation où un azéotrope de faible point d'ébullition composé du solvant et de l'alcool est retiré de l'ester d'acide carboxylique.

5. Procédé selon l'une quelconque des revendications 2 à 4, caractérisé en ce que le solvant rendant azéotrope est une paraffine ayant un point d'ébullition de 30 à 150°C.

6. Procédé selon la revendication 5, caractérisé en ce que la paraffine est de l'octane.

7. Procédé selon l'une quelconque des revendications 2 à 6, caractérisé en ce que le catalyseur est $BF_3 \cdot CH_3OH$ et le produit obtenu est de l'isobutyrate de méthyle, si l'oléfine est du propylène ou du propionate de méthyle si l'oléfine est de l'éthylène.

8. Procédé selon l'une quelconque des revendications précédentes, caractérisé en ce que le catalyseur est $BF_3 \cdot H_2O$ ou $BF_3 \cdot CH_3OH$ et est formé par réaction de 0.75 à 2 moles de $BF_3$ avec une mole de $CH_3OH$.

9. Procédé selon l'une quelconque des revendications précédentes, caractérisé en ce que le produit de la réaction de carbonylation est deshydrogéné en un acide carboxylique insaturé ou ester d'acide carboxylique insaturé correspondant.

10. Procédé pour la carbonylation d'une oléfine par réaction de l'oléfine avec de l'oxyde de carbone en présence d'un catalyseur, caractérisé en ce que l'oléfine est de l'éthylène ou du propylène et le catalyseur contient des quantités équimolaires de $BF_3$ et d'un alcool, et la réaction étant entreprise à une température de 0 à 100°C, ainsi le produit obtenu est un ester de carboxylate où le groupe carboxylate a un atome de carbone de plus que l'oléfine employée, la réaction étant effectuée jusqu'à ce qu'environ 50% de l'alcool dans ledit catalyseur soient consommés pour former un ester d'acide carboxylique et libérer $BF_3$ libre; suffisamment de $BF_3$ se sépare de la masse réactionnelle pour laisser un premier résidu contenant des quantités équimolaires de $BF_3$, de l'alcool et de l'ester d'acide carboxylique; le premier résidu est mélangé à de l'alcool supplémentaire; ensuite ledit mélange est distillé pour séparer en tête un azéotrope dudit alcool et dudit ester d'acide carboxylique jusqu'à ce qu'il reste un second résidu contenant $BF_3$ à l'alcool à un rapport molaire de 1:2; et ensuite ledit second résidu est combiné à du $BF_3$ supplémentaire pour former le complexe catalytique.